# EUROPÄISCHE PATENTSCHRIFT

(11) **EP 2 594 630 B1**
(45) Veröffentlichungstag und Bekanntmachung des Hinweises auf die Patenterteilung: **30.12.2020**
(21) Anmeldenummer: 12007794.6
(22) Anmeldetag: 19.11.2012
(51) Int. Cl.: C12M 1/00, G01N 33/50

(54) **Verteilervorrichtung für eine Probenexpositionsanordnung**
Distributor device for a sample exposure assembly
Dispositif de répartition pour un agencement d'exposition d'échantillons

(30) Priorität: 20.11.2011 DE 102011118958
(43) Veröffentlichungstag der Anmeldung: 22.05.2013
(73) Patentinhaber: Pieter Van Weenen & Co. GmbH The House of Innovation, 79183 Waldkirch (DE)
(72) Erfinder: Krebs, Tobias, 79261 Gutach (DE)
(74) Vertreter: von Pichler, Cletus

(56) Entgegenhaltungen:
- WO-A1-01/70927
- US-A1- 2002 164 653
- US-A1- 2010 273 246

## Beschreibung

### Gebiet der Erfindung

Die vorliegende Erfindung betrifft im Allgemeinen Vorrichtungen, um eine Probe einer luftgetragenen Mischung zu exponieren.

### Hintergrund der Erfindung

Begasungsvorrichtungen, auch Kultur- oder Expositionsvorrichtungen genannt, werden verwendet, um einer Probe (z.B. Zell- oder Bakterienkultur, chemische Testsubstanz) mit einem Fluid zu beaufschlagen und zu überprüfen, welche Wirkungen das Fluid auf die Probe hat.

Als Fluide kommen beispielsweise Gase, Gasmischungen, luftgetragene Substanzen, luftgetragene Mischungen (z.B. Rauch von Rauchwaren, wie Zigaretten und Zigarren), Aerosole, Umweltschadstoffe, medizinische Substanzen, Abgase, wie z.B. Verbrennungsabgase, luftgetragene Partikel, etc. in Frage.

Bei einer Begasungsvorrichtung sind üblicherweise mehrere Probenbehälter jeweils mit einer Probe vorgesehen, denen aus einer gemeinsamen Fluidquelle Fluid zugeführt wird. Als Quelle kann beispielsweise eine das Fluid erzeugende Vorrichtung (z.B. Rauchmaschine, Aerosolgenerator, Motorenprüfstand, Förderanlagen, wie z.B. Förderpumpen für Luft, etc.) oder eine Speichervorrichtung für das Fluid dienen (z.B. Gasflaschen, Fluidsammelbehälter).

Das den Proben zuzuführenden Fluid kann z.T. auch zunächst verdünnt (z.B. mit Umgebungsluft, Sauerstoff, synthetisierte Luft, Pressluft, etc.) und/oder mit einem oder mehreren weiteren Fluiden (z.B. Schadstoffe) angereichert werden, bevor es den Probenbehältern zugeführt wird.

Bei Verwendung einer gemeinsamen Fluidquelle ist im Allgemeinen eine Verteilervorrichtung zwischen der Fluidquelle und den Probenbehältern erforderlich.

### Druckschriftlicher Stand der Technik

Die US 2017/0273246 A1 offenbart eine Verteilervorrichtung für eine Expositionsanordnung, bei der mittels einer Fluidauslasseinrichtung, die einem Probenbehälter zugeordnet ist, Fluid zugeführt wird. Fluidauslasseinrichtung weist Fluidauslasseinrichtungs-Einlass auf, der von einer Fluid-Verteilungseinrichtung Fluid, welches dem Probenbehälter zugeführt werden soll, erhält und über einen Fluidauslasseinrichtungs-Auslass das Fluid dem Probenbehälter zuführt. Die WO 01/70927 A1 offenbart eine Verteilervorrichtung für eine Expositionsanordnung. Die Verteilervorrichtung umfasst eine Verwirbelungskammer, um Probenbehältern zuzuführendes Fluid zuvor zu verwirbeln.

Die US 2002/164653 A1 offenbart eine Verteilervorrichtung für eine Expositionsanordnung. Die Verteilervorrichtung umfasst ein Abgaberohr das mehrere Auslässe aufweist, die jeweils einem Probenbehälter zugeordnet sind. Das Abgaberohr weist ebenfalls einen Auslass auf, der einem Auffangbehälter zugeordnet ist.

### Aufgabe der Erfindung

Aufgabe der vorliegenden Erfindung ist es, Lösungen bereitzustellen, die eine gegenüber bekannten Ansätzen verbesserte Verteilung von mehreren Probenbehältern zuzuführendem Fluid ermöglicht.

### Kurzbeschreibung der Erfindung

Zur Lösung der obigen Aufgabe stellt die vorliegende Erfindung eine Verteilervorrichtung gemäß dem unabhängigen Anspruch bereit. Bevorzugte Ausführungsformen sind in den abhängigen Patentansprüchen angegeben. Insbesondere sieht die vorliegende Erfindung eine Verteilervorrichtung für eine Probenexpositionsanordnung vor, die wenigstens zwei Probenbehälter und eine Fluidquelle für den Probenbehältern zuzuführendes Fluid aufweist. Die erfindungsgemäße Verteilervorrichtung umfasst eine Verteilungseinrichtung mit einem Verteilungseinrichtungs-Einlass, um Fluid der Verteilervorrichtung zuzuführen. Der Verteilungseinrichtungs-Einlass kann beispielsweise mit einem Fluideinlass der Verteilungseinrichtungs-Einlass verbunden sein oder damit in Fluidverbindung stehen. Die Verteilungseinrichtung weist ferner jeweils einen Verteilungseinrichtungs-Auslass pro Probenbehälter und eine Verteilungskammer zwischen dem Verteilungseinrichtungs-Einlass und den Verteilungseinrichtungs-Auslässen auf. Die Verteilervorrichtung umfasst des Weiteren wenigstens eine Fluidauslasseinrichtung pro Verteilungseinrichtungs-Auslass. Die wenigstens eine Fluidauslasseinrichtung weist jeweils einen Fluidauslasseinrichtungs-Einlass, der mit dem entsprechenden Verteilungseinrichtungs-Auslass in Fluidverbindung steht, einen ersten Fluidauslasseinrichtungs-Auslass zur Abgabe von Fluid in den entsprechenden Probenbehälter, und einen zweiten Fluidauslasseinrichtungs-Auslass zur Abgabe von überschüssigem Fluid auf.

Die Verteilungseinrichtung kann zwischen der Verteilungskammer und den Verteilungseinrichtungs-Auslässen jeweils wenigstens eine Fluidführung aufweisen.

Die Verteilungskammer kann im Wesentlichen kugelförmig oder halbkugelförmig sein.

In der Verteilungskammer kann eine strömungsbeeinflussende Einrichtung angeordnet sein.

An einer Innenseite der Verteilungskammer kann für jede Fluidführung eine Fluidleitausnehmung ausgebildet sein.

Die Verteilungseinrichtung kann eine abnehmbare Abdeckung umfassen, die wenigstens einen Teil der Verteilungskammer definiert.

Es können die Verteilungskammer wenigstens teilweise und/oder die Fluidführungen in einem ersten Körper der Verteilungseinrichtung ausgebildet sein.

Die Fluidführungen können wenigstens teilweise in einem zweiten Körper ausgebildet sein.

Der erste Körper und der zweite Körper können als separate Komponenten vorgesehen sein, die verbindbar sind.

Der erste Körper und der zweite Körper können jeweils komplementäre die Fluidführungen bildende Ausnehmung aufweisen.

Die Fluidführung im Wesentlichen die gleichen Abmessungen haben und/oder wenigstens eine der Fluidführungen kann gekrümmt verlaufen.

Die Fluidauslasseinrichtungs-Auslässe können zur Abgabe von überschüssigem Fluid mit einem Überschussfluidbehälter in Fluidverbindung stehen.

Der Überschussfluidbehälter kann einen Auslass zur Abgabe von darin vorhandenem Fluid haben

Die Verteilervorrichtung kann eine Fluidzufuhr-Selektionseinrichtung aufweisen, die mit den zweiten Fluidauslasseinrichtungs-Auslässen und mit dem Verteilungseinrichtungs-Einlass in Fluidverbindung steht und ausgelegt ist, entweder Fluid der Fluidquelle oder Fluid von den zweiten Fluidauslasseinrichtungs-Auslässen oder eine Mischung von Fluid der Fluidquelle und Fluid von zweiten Fluidauslasseinrichtungs-Auslässen dem Verteilungseinrichtungs-Einlass zuzuführen.

Die Verteilervorrichtung kann eine Fluidvolumenausgleichseinrichtung aufweisen, die mit den zweiten Fluidauslasseinrichtungs-Auslässen in Fluidverbindung steht.

Ferner sieht die vorliegende Erfindung eine Probenexpositionsanordnung mit einer erfindungsgemäßen Verteilervorrichtung vor.

Die Probenexpositionsanordnung kann wenigstens zwei Probenbehältern und/oder eine Fluidquelle für den Probenbehältern zuzuführendes Fluid aufweisen.

### Kurzbeschreibung der Zeichnungen

Bevorzugte Ausführungsformen der vorliegenden Erfindung werden im Folgenden unter Bezugnahme auf die beigefügten Zeichnungen erläutert, die zeigen:
- Fig. 1: eine schematische Darstellung einer Probenexpositionsanordnung mit einer Verteilervorrichtung und einer Probenbehälteranordnung,
- Fig. 2: schematische Querschnittsdarstellungen einer Ausführungsform einer Verteilervorrichtung,
- Fig. 3: schematische Darstellungen von Ausführungsformen von Verteilungskammern für Verteilervorrichtungen,
- Fig. 4: schematische Querschnittsdarstellungen von Ausführungsformen von Fluidauslasseinrichtungen,
- Fig. 5: schematische Querschnittsdarstellungen einer Ausführungsform einer 3-fachen Verteilervorrichtung,
- Fig. 6: schematische Querschnittsdarstellungen einer Ausführungsform einer 4-fachen Verteilervorrichtung,
- Fig. 7: eine schematische Darstellung einer Probenexpositionsanordnung mit optionaler Fluidrückführung,

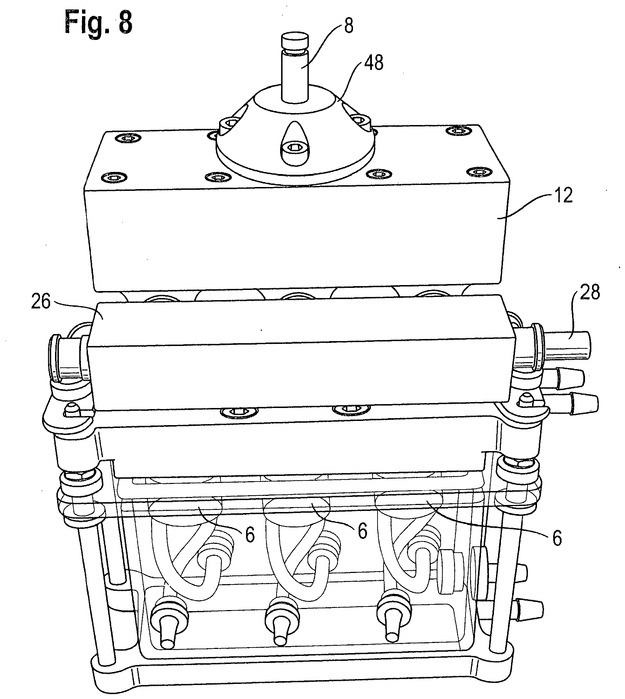

- Fig. 8: eine Darstellung einer Probenexpositionsanordnung mit einer Verteilervorrichtung,

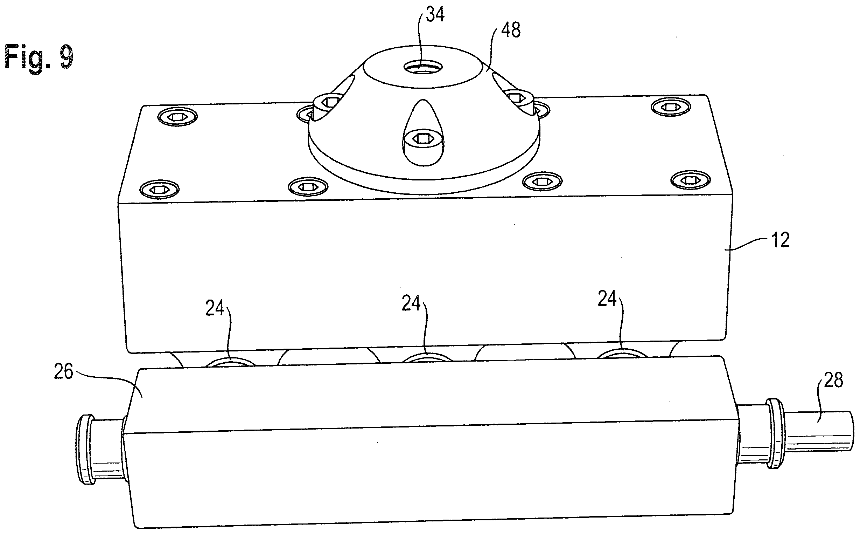

- Fig. 9: eine perspektivische Darstellung der Verteilervorrichtung von Fig. 8,

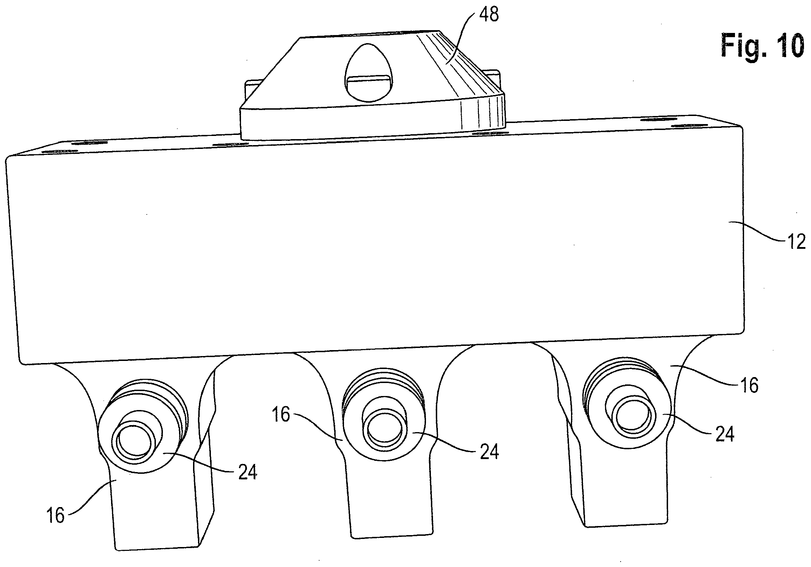

- Fig. 10: eine Darstellung der Verteilervorrichtung von Fig. 8 ohne Überschussfluidbehälter,

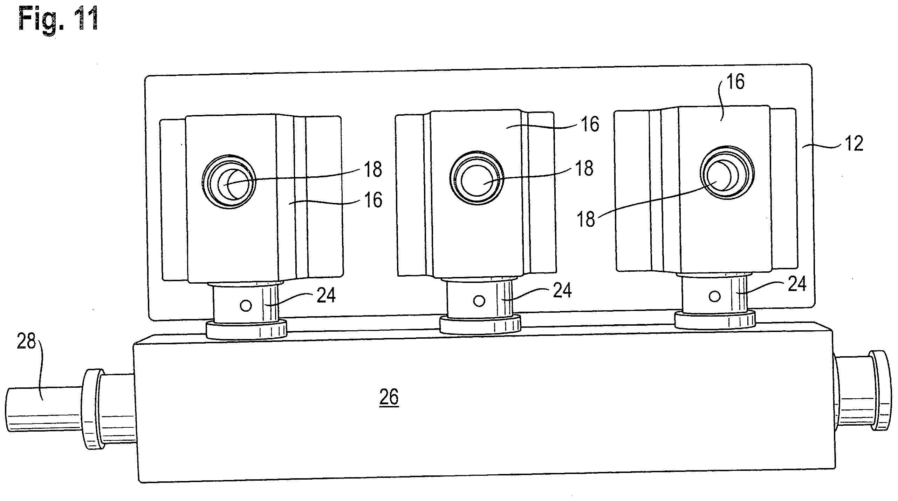

- Fig. 11: eine Darstellung der Abgabeseite der Verteilervorrichtung von Fig. 8, und

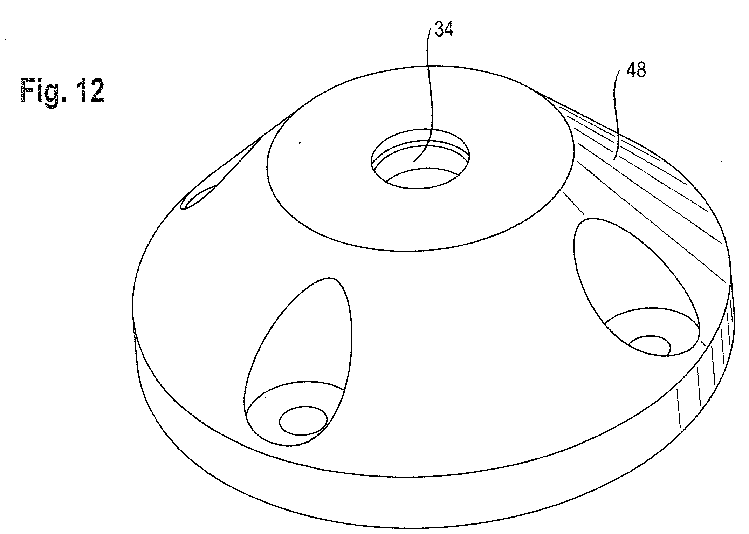

- Fig. 12: eine Darstellung eines Deckels für die Verteilungseinrichtung der Verteilervorrichtung von Fig. 8.

### Beschreibung bevorzugter Ausführungsformen

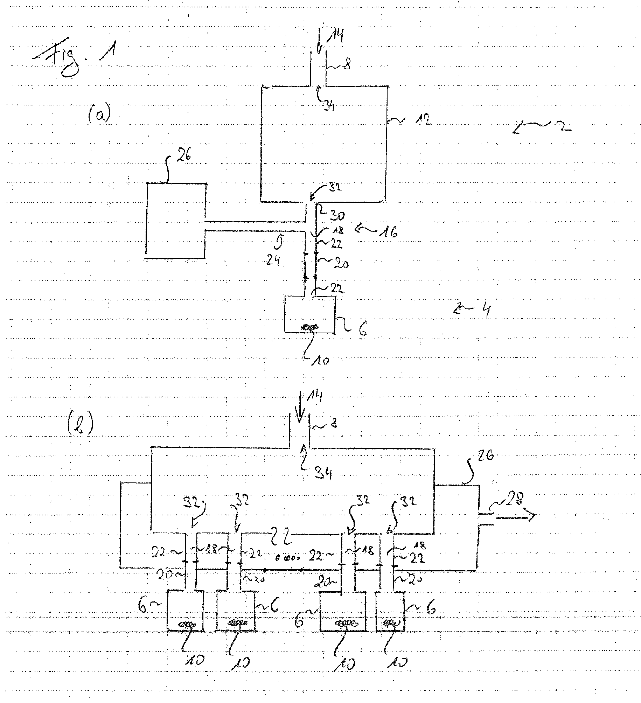

Fig. 1 zeigt eine Probenexpositionsanordnung mit einer im Ganzen mit 2 bezeichneten Verteilervorrichtung und einer Anordnung 4 mit Probenbehältern 6. Beim Betrieb der Probenexpositionsanordnung wird über einen Fluideinlass 8 der Verteilervorrichtung 2 Fluid (z.B. eine luftgetragene Mischung), das von einer Fluidquelle bereitgestellt wird, zugeführt, mittels der Verteilereinrichtung 2 verteilt und den Probenbehältern 6 und darin angeordneten Proben 10 zugeführt.

Die Verteilervorrichtung 2 umfasst eine Verteilungseinrichtung 12, der eingangsseitig über den Fluideinlass 8 Fluid zugeführt werden kann, was durch den Pfeil 14 veranschaulicht ist. Der Fluideinlass 8 mit z.B. mittels einer oder mehreren Reduzierhülsen, Adaptern oder dergleichen im Querschnitt eine Fluidzuleitung von der Fluidquelle angepasst werden. In der Verteilungseinrichtung 12 wird, wie im Folgenden näher erläutert, zugeführtes Fluid verteilt und Fluidauslasseinrichtungen 16 zugeführt.

Über erste Fluidauslasseinrichtungs-Auslässe 18 der Fluidauslasseinrichtungen 16 kann Fluid in die Probenbehälter 6 eingebracht werden. Hierzu können die ersten Fluidauslasseinrichtungs-Auslässe 18 z.B. mittels einer Schlauch- oder Rohrverbindungen 20 mit den Einlässen 22 der Probenbehältern 6 verbunden sein, oder direkt auf die zu den Probenbehältern führenden Einlassrohre aufgesteckt werden.

Die Anzahl der Fluidauslasseinrichtungen 16 entspricht bei der gezeigten der Anzahl der Probenbehälter 6 und kann, wie in Fig. 1 (b) die durch die Punkte zwischen den Fluidauslasseinrichtungen 16 angegeben, ebenso wir die Zahl der Probenbehälter 6 im Wesentlichen frei gewählt werden. So sind beispielsweise Ausführungsformen mit zwei, drei, vier, etc. Fluidauslasseinrichtungen-16 und Probenbehältern 6 vorgesehen, die wie bei der gezeigten Ausführungsform in einer Reihe angeordnet sind. Bei weiteren Ausführungsformen können mehrere nebeneinander in Reihe von zwei und mehr Fluidauslasseinrichtungen 16 angeordnete bzw. Probenbehälter 6 verwendet werden. Ferner können mehr als eine Fluidauslasseinrichtung 16 pro Probenbehälter 6 vorgesehen sein. Auch ist es möglich, als Beispiel zum Erleichtern des Verständnisses der Erfindung, mit einer Fluidauslasseinrichtung 16 mehr als einen Probenbehälter 6 zu versorgen.

Die Fluidauslasseinrichtungen 16 weisen ferner zweite Fluidauslasseinrichtungs-Auslässe 24 auf, über die ebenfalls Fluid abgeführt werden kann, allerdings nicht zu Probenbehältern 6. Vielmehr wird über die zweiten Fluidauslasseinrichtungs-Auslässe 24 Fluid, das nicht den Probenbehältern 6 zugeführt werden soll und/oder kann, abgeführt.

Solches im Folgenden auch als Fluidüberschuss oder überschüssiges Fluid bezeichnetes Fluid kann auftreten, wenn z.B. die Menge an der Verteilervorrichtung 2 zugeführtem Fluid höher als die zur Exposition der Proben 10 benötigte Fluidmenge ist. So ist es beispielsweise möglich, dass der Verteilervorrichtung 2 etwa drei Liter Fluid pro Minute zugeführt wird, den Probenbehältern 6 jeweils aber nur etwa 5 Milliliter pro Minute (i.e. bei 3 Probenbehältern insgesamt 15 Milliliter pro Minute). Dies kann z.B. darauf zurückzuführen sein, dass Fluid in die Probenbehältern 6 hinein angesaugt wird, was die Fluidzufuhr steuert und eben auch begrenzt.

Ein Vorteil der Entfernung von Fluidüberschuss kurz vor den Probenbehältern 6 besteht darin, dass den Probenbehältern 6 zuzuführendes Fluid (und damit dessen Bestandteile, z.B. Partikel) deutlich länger als bei bekannten Ansätzen und bis zuletzt (d.h. im Wesentlichen bis unmittelbar vor die Probenbehälter 6) mit einer höheren Geschwindigkeit transportiert werden. Dies führt zu geringeren Verlusten, die auftreten können, wenn das Fluid (und damit dessen Bestandteile, z.B. Partikel) über einen längeren Weg mit niedrigerer Flussrate (z.B. 5 Milliliter pro Minute) transportiert werden.

In solchen Fällen wird überschüssiges Fluid über die zweiten Fluidauslasseinrichtungs-Auslässe 24 entfernt und kann einem optionalen Überschussfluidbehälter 26 zugeführt werden. Der Überschussfluidbehälter 26 selbst kann zum Sammeln überschüssigen Fluids (wenigstens teilweise) dienen und/oder über einen Überschussfluidbehälter-Auslass 28 mit einer separaten Sammelvorrichtung (z.B. zur späteren Entsorgung) verbunden sein. Einer solchen separaten Sammelvorrichtung kann überschüssiges Fluid auch direkt von den zweiten Fluidauslasseinrichtungs-Auslässen 24 zugeführt werden. Ferner ist es vorgesehen, wie weiter unten detaillierter erläutert, überschüssiges Fluid über die zweiten Fluidauslasseinrichtungs-Auslässe 24 (mit oder ohne Überschussfluidbehälter 26) zurück zum Fluideinlass 8 zu führen. Ergänzend oder optional kann überschüssiges Fluid (auch) zur weiteren Verwendung zu einer separaten Versuchsanordnung (z.B. eine weitere, nachgelagerte Probenexpositionsanordnung) überführt werden.

Der Überschussfluidbehälter 26 kann lösbar mit den Fluidauslasseinrichtungs-Auslässen 24 verbunden sein. Dies ermöglicht es beispielsweise, den Überschussfluidbehälter 26 zusammen mit darin gesammeltem Fluid zu entfernen. Der Überschussfluidbehälter 26 kann auch wie ein Auspuff z.B. eines Kraftfahrzeug wirken, nämlich beim Zuführen von Fluid in die Probenbehälter 6 entstehende Schwankungen im Fluidstrom zu reduzieren. Schwankungen im Fluidstrom können insbesondere dann auftreten, wenn zur Fluidzufuhr in die Probenbehälter 6 in diesen Unterdruck erzeugt wird, um Fluid z.B. bestimmter Menge pro Zeiteinheit anzusaugen.

Die Fluidauslasseinrichtungen 16 sind über ihre Fluidauslasseinrichtungs-Einlässe 30 mit Verteilungseinrichtungs-Auslässen 32 der Verteilungseinrichtung 12 verbunden. Die Verteilungseinrichtungs-Auslässe 32 sind wie die Probenbehälter 6 und die Fluidauslasseinrichtungen 16 in einer Reihe angeordnet. Die Anordnung der Verteilungseinrichtungs-Auslässe 32 in Reihe ermöglicht die Verwendung von in wenigstens einer Reihe, linear angeordneten Probenbehältern 6. Bei bekannten Anordnungen werden oftmals kreisförmig angeordnete Probenbehälter verwendet, was es erschweren kann die Anordnung durch zusätzliche Probenbehälter zu erweitern. Dies ist bei einer linearen Probenbehälteranordnung, wie von der vorliegenden Erfindung vorgesehen, nicht der Fall. Außerdem ist bei einer kreisförmigen Anordnung der Platzbedarf größer. Mit einer linearen Anordnung lassen sich z.B. sechs Probenbehälter mit jeweils unterschiedlichen Fluiden beschicken.

Des Weiteren ist es vorgesehen, die Verteilungseinrichtung 12 so auszugestalten, dass sich die Führungen (z.B. Kanäle) in der Verteilungseinrichtung 12 beginnend beim Verteilungseinrichtungs-Einlass 34 bis zu den Verteilungseinrichtungs-Auslässen 32 wenigstens im Wesentlichen entsprechen. Dies kann beispielsweise erreicht werden, indem die Fluidführungen in der Verteilungseinrichtung 12 gleich dimensioniert (z.B. gleich lang, gleicher Durchmesser) sind. Ein Aspekt der vorliegenden Erfindung besteht darin, derartige gleiche Fluidführungen in der Verteilungseinrichtung 12 so auszuführen, die es ermöglicht, die Verteilungseinrichtungs-Auslässe 32 in einer Reihe, linear anzuordnen. Dies wird weiter unter detaillierter beschrieben.

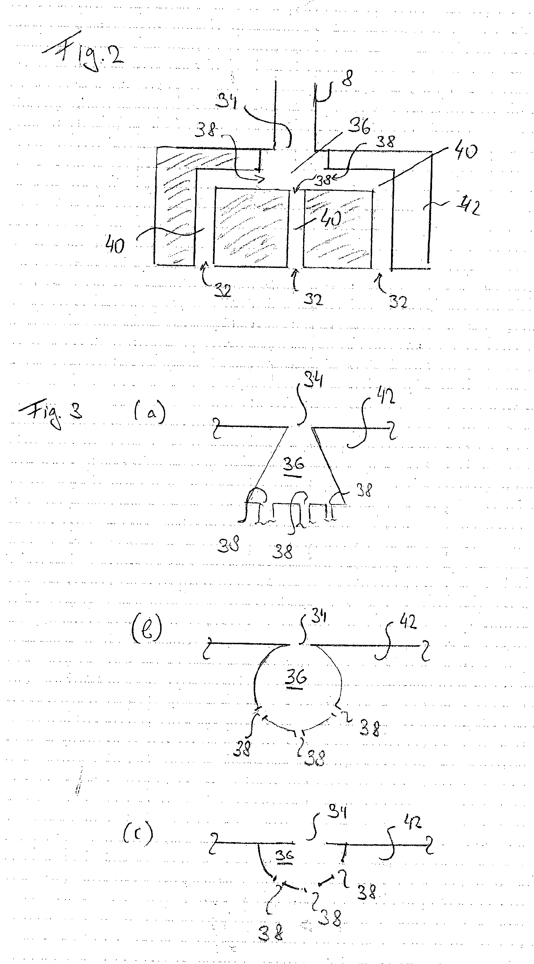

Fig. 2 veranschaulicht schematisch den Aufbau einer Verteilungseinrichtung 12. Über den Verteilungseinrichtungs-Einlass 34 erhaltenes Fluid wird einer Verteilungskammer 36 zugeführt. Die Verteilungskammer 36 weist Auslassöffnungen 38 auf, die zu Fluidführungen 40 führen, die wiederum in den Verteilungseinrichtungs-Auslässen 32 enden. Die Anzahl der Auslassöffnungen 38, Fluidführungen 40 bzw. Verteilungseinrichtungs-Auslässe 32 kann davon abhängen, wie oft Fluid aufgeteilt werden soll. Dabei ist es vorgesehen, für jeden Probenbehälter 6 eine Fluidaufteilung vorzunehmen und dementsprechend eine Anzahl an Auslassöffnungen 38, Fluidführungen 40 bzw. Verteilungseinrichtungs-Auslässen 32 zu verwenden, die der Anzahl an Probenbehältern 6 entspricht. Es möglich, für einen oder mehrere Probenbehälter 6 mehr als eine Fluidaufteilung vorzunehmen und dementsprechend für diese Probenbehälter 6 jeweils mehr als zwei Auslassöffnungen 38, Fluidführungen 40 bzw. Verteilungseinrichtungs-Auslässe 32 zu verwenden. In solchen Fällen ist es möglich, wenn für einen Probenbehälter 6 mehr als zwei Verteilungseinrichtungs-Auslässe 32 vorgesehen, diese jeweils mit einer eigenen Fluidauslasseinrichtung 16 zu verbinden, oder mehreren oder allen einem Probenbehälter 6 zugeordneten Verteilungseinrichtungs-Auslässen eine gemeinsame Fluidauslasseinrichtung 16 zuzuordnen.

Die Fluidführungen 40 sind bei der Ausführungsform von Fig. 2 als gewinkelt unterschiedliche lange Kanäle veranschaulicht. Bei weiteren Ausführungsformen sind die Fluidführungen 40 alle im Wesentlichen gleich lang und/oder haben den gleichen Querschnitt. Ferner können die Fluidführungen 40 gekrümmt verlaufen, insbesondere so, dass gleichmäßige, verwirbelungsfreie, etc. Fluidströmungen erreicht werden.

Bei der Ausführungsform von Fig. 2 sind der Verteilungseinrichtungs-Einlass 34, die Verteilungskammer 36, dessen Auslassöffnungen 38 sowie die Fluidführungen 40 und Verteilungseinrichtungs-Auslässe 32 in einem Körper 42 ausgebildet. Der Körper 42 kann wie gezeigt einstückig ausgebildet sein und beispielsweise u.a. aus Kunststoff, Metall, Keramik und/oder Glas hergestellt sein. Der Körper 42 ist als einstückiger Körper gezeigt, kann aber, wie weiter unten erläutert, auch mehrstückig aufgebaut sein. Im Folgenden wird zunächst auf den Körper 42 Bezug genommen. Diesbezüglich Ausführungen gelten aber auch entsprechende anders ausgeführte Komponenten, wie z.B. ein weiter unten beschriebener erster Körper der Verteilungseinrichtung.

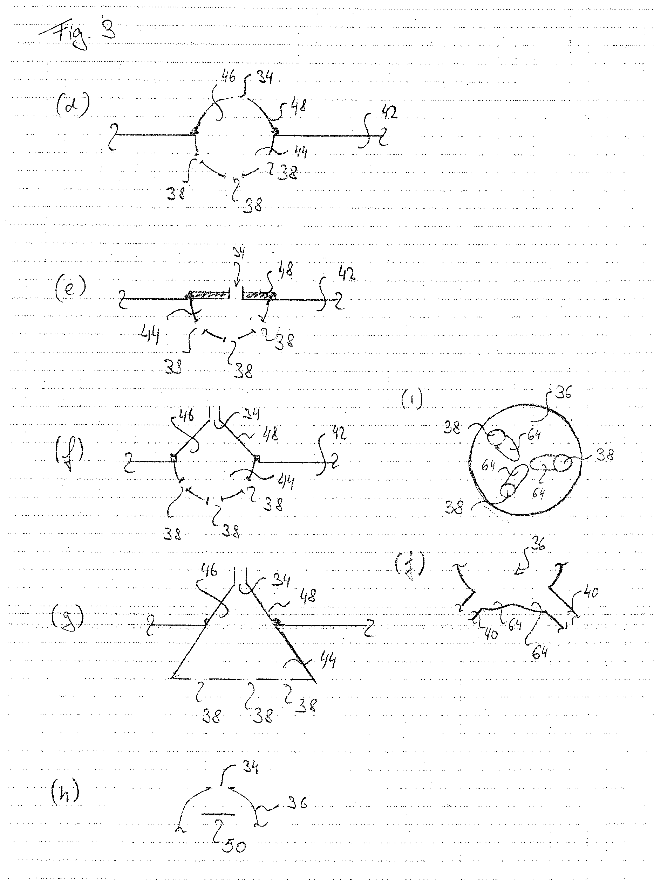

Fig. 3 zeigt mehrere Ausführungsformen von Verteilungskammern 36 der Verteilungseinrichtung 12. Die in Fig. 3 (a), (b) und (c) veranschaulichten pyramiden- oder kegelstumpfförmigen, kugelförmigen bzw. halbkugelförmigen Verteilungskammern 36 sind in dem Körper 42 ausgebildet.

Die in Fig. 3 (d), (e), (f) und (g) gezeigten, beispielhaften Verteilungskammern 36 umfassen einen ersten Verteilungskammerteil 44 , der in dem Körper 42 ausgebildet ist, und einen zweiten Verteilungskammerteil 46, der von einer Abdeckung 48 definiert wird. Die Abdeckung 48 ist mit dem Körper 42 lösbar verbunden, z.B. mittels Schraub- und/oder Steck-/Rast-/Schnappverbindungen. Derartige Ausführungsformen können es erleichtern, auf Verteilungskammern 36 zuzugreifen, um beispielsweise diese zu reinigen. Auch können bei solchen Ausführungsformen durch Kombination eines Typs Verteilungskammerteils 44 mit unterschiedlichen Abdeckungen 48 ohne sonstige Modifikation der Gesamtanordnung unterschiedliche Verteilungskammern 36 erreicht werden. So kann die Verteilungskammerteil 44 der Fig. 3 (d) und (e), die einander entsprechen, mit der Abdeckung 48 der Fig. 3 (d) kombiniert werden, um eine kugelförmigen Verteilungskammer 36 zu erhalten, und mit der Abdeckung 48 der Fig. 3 (e), um eine halbkugelförmige Verteilungskammer 36 zu erhalten.

Ferner erlauben es solche Ausführungsformen, unterschiedliche erste und zweite Verteilungskammerteile zu kombinieren. Beispielsweise kann die Abdeckung 48 der Fig. 3 (d) mit dem ersten Verteilungskammerteil 44 der Fig. 3 (f) oder (g) zu kombinieren.

Wie in Fig. 3 (h) veranschaulicht kann, unabhängig von der Form der Verteilungskammer 36, darin ein strömungsbeeinflussendes Mittel 50 angeordnet sein, wie z.B. eine Prallfläche, Drallkörper und/oder ein Verwirbelungsbauteil.

Fig. 3 (i) und (j) zeigen eine weitere Variante einer Verteilungskammer 36. In Fig. 3 (i) ist eine Ansicht in die Verteilungskammer 36 hinein in Strömungsrichtung veranschaulicht, während Fig. 3 (j) eine Querschnittsansicht zeigt. Bei der gezeigten Ausführungsform sind drei zu Fluidführungen 40 führende Auslassöffnungen 38 gezeigt. Den Auslassöffnungen 38 ist jeweils eine Fluidleitausnehmung 64 zugeordnet. Die Fluidleitausnehmungen 64 sind dort, wo sie mit der jeweiligen Auslassöffnung 38 zusammentreffen oder an diese angrenzen, am tiefsten und werden in Richtung auf die Mitte hin flacher. Die Fluidleitausnehmungen 64 können die Verteilung von Fluid zu den Auslassöffnungen 38 und somit zu den Fluidführungen 40 und letztendlich zu den Probenbehältern 6 verbessern.

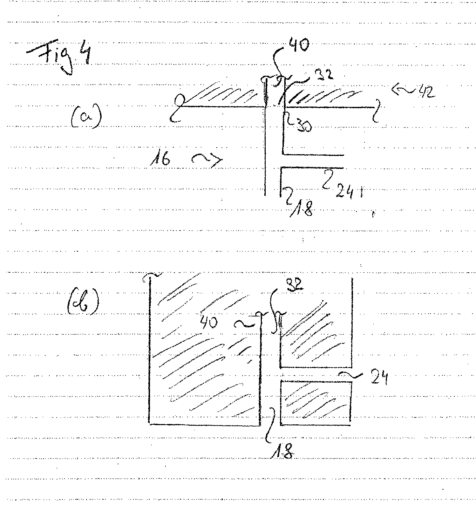

Fig. 4 veranschaulicht Ausführungsformen, Fluidauslasseinrichtungen 16 zu gestalten. Bei der Variante von Fig. 4 (a) ist die Fluidauslasseinrichtung 16 außerhalb des Körpers 42 angeordnet, während bei der Variante von Fig. 4 (b) die Fluidauslasseinrichtung 16 im Körper 42 angeordnet ist.

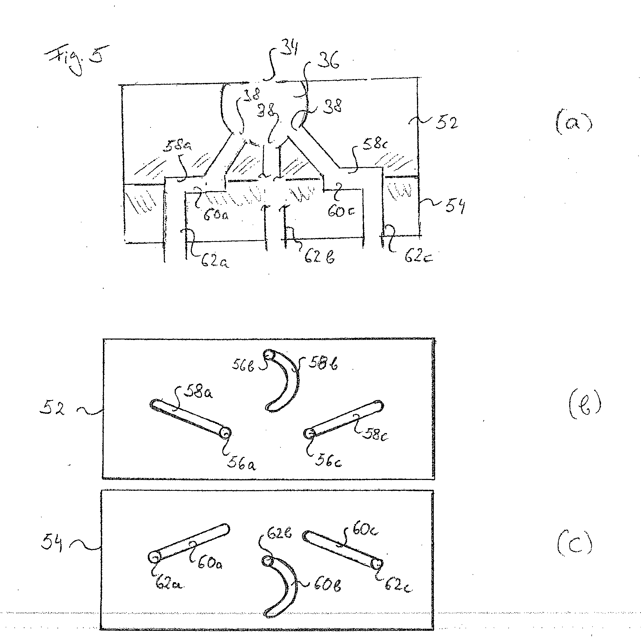

Fig. 5 zeigt eine Ausführungsform der Verteilungseinrichtung 12 mit beispielhaft gewählten drei Fluidführungen 40, die jeweils einen Probenbehälter versorgen sollen. Der Körper 42 der Verteilungseinrichtung 12 dieser Ausführungsform umfasst einen ersten Körper 52 und einen zweiten Körper 54. Die Körper 52 und 54 können z.B. abhängig vom Herstellungsverfahren einstückig ausgebildet sein. Daher kann man in solchen Fällen die Körper 52 und 54 auch als Körperteile des Körpers 42 bezeichnen. Ferner können die Körper 52 und 54 jeweils wiederum ein-, zwei- oder mehrstückig sein, beispielsweise ebenfalls abhängig vom Herstellungsverfahren. Der erste Körper 52 und der zweite Körper 54 können beispielsweise u.a. aus Kunststoff, Metall, Keramik und/oder Glas hergestellt sein, wobei die Körper 52 und 54 auch unterschiedlich aufgebaut sein können, und/oder mittels Schraub- und/oder Steck-/Rast-/Schnappverbindungen miteinander verbunden sein. Auch können die Körper 52, 54 und 42 insgesamt einstückig ausgeführt sein.

Bei der Variante von Fig. 5 sind die Fluidführungen 40 gleich dimensioniert, und haben insbesondere die gleiche Länge und im Wesentlichen überall den gleichen Querschnitt. Um dies in Verbindung damit zu erreichen, dass die Verteilungseinrichtungs-Auslässe 23 in einer Reihe angeordnet sind, wird wie folgt erreicht. Die Fluidführungen 40 verlaufen beginnend bei den Auslassöffnungen 38 der Verteilungskammer 36 zunächst im Wesentlichen geradlinig, beispielsweise in Form von Bohrungen. Diese Bereiche der Fluidführungen 40 enden bzw. öffnen sich bei den in Fig. 5 (a) mit 56a, 56b und 56c gekennzeichneten Stellen, die auch in Fig. 5 (b) bezeichnet sind, und gehen dort in Ausnehmungen 58a, 58b und 58c im ersten Körper 52 über.

Die Ausnehmungen 58a, 58b und 58c sind, wie in Fig. 5 (b) veranschaulicht, in der gemäß Fig. 5 (a) unteren Seite des ersten Körpers 52 ausgeformt. Die Ausnehmungen 58a, 58b und 58c sind so ausgeformt, dass, wenn der erste Körper 52 und der zweite Körper 54 miteinander verbunden sind, mit im Folgenden beschriebenen im zweiten Körper ausgeformten Ausnehmungen 60a, 60b und 60c jeweils einen Teil der entsprechenden Fluidführung 40 bilden.

Die Ausnehmungen 60a, 60b und 60c sind, wie in Fig. 5 (c) veranschaulicht, in der gemäß Fig. 5 (a) oberen Seite des zweiten Körpers 54 ausgeformt. Die Ausnehmungen 60a, 60b und 60c enden in Strömungsrichtung betrachtet bei Durchgängen 62a, 62b und 62c, die durch den zweiten Körper 54 zu den Fluidauslasseinrichtungs-Einlässen 30, von aus Fluid zu den Verteilungseinrichtungs-Auslässen 32 gelangen kann.

Durch die bezüglich der horizontalen Darstellungsebene schräg verlaufenden durch die Ausnehmungen 58a/60a und 58c/60c gebildeten Bereiche der linken und rechten Fluidführungen 40 und durch den bogenartigen Verlauf des durch die Ausnehmungen 58b/60b gebildeten Bereichs der mittleren Fluidführung 40 wird erreicht, dass einerseits die Fluidführungen 40 im Wesentlichen gleich dimensioniert sind, die gleiche Länge und im Wesentlichen überall den gleichen Querschnitt haben, und andererseits die Verteilungseinrichtungs-Auslässe 32 in einer Reihe angeordnet sind. Letzteres in Verbindung mit einer korrespondierenden linearen Anordnung der Fluidauslasseinrichtungen 16 und deren ersten Fluidauslasseinrichtungs-Auslässen 18 können ebenfalls linear in einer Reihe angeordnete Probenbehälter versorgt werden.

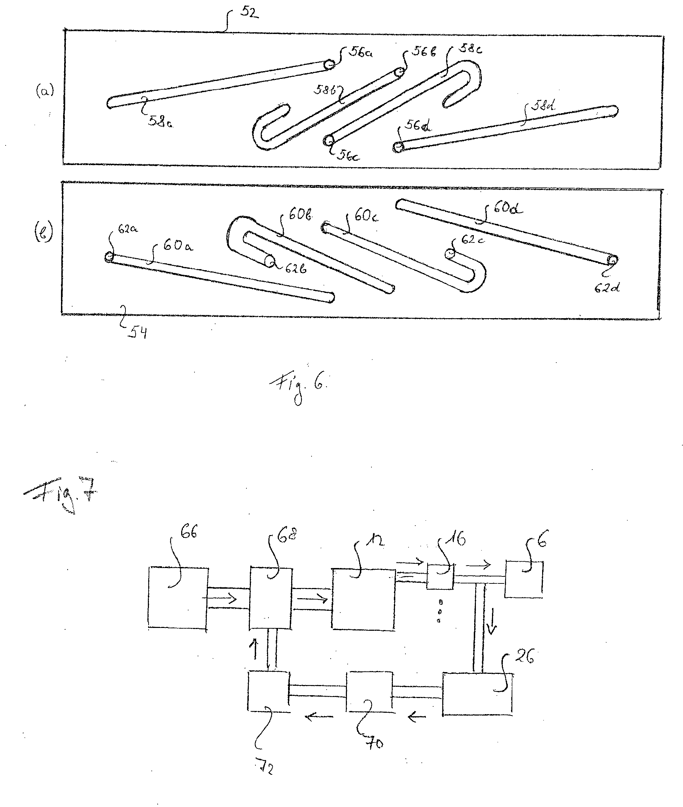

Fig. 6 zeigt eine Ausführungsform der Verteilungseinrichtung 12 mit beispielhaft gewählten vier Fluidführungen 40, die jeweils einen Probenbehälter versorgen sollen. Soweit im Folgenden nicht anders angegeben, gelten die obigen Ausführungen, insbesondere hinsichtlich der Fig. 5, auch für die Variante der Fig. 6.

Der Körper 42 der Verteilungseinrichtung 12 dieser Ausführungsform umfasst einen ersten Körper 52 und einen zweiten Körper 54. Die Körper 52 und 54 können z.B. abhängig vom Herstellungsverfahren einstückig ausgebildet sein. Daher kann man in solchen Fällen die Körper 52 und 54 auch als Körperteile des Körpers 42 bezeichnen. Ferner können die Körper 52 und 54 jeweils wiederum ein-, zwei- oder mehrstückig sein, beispielsweise ebenfalls abhängig vom Herstellungsverfahren. Auch können die Körper 52, 54 und 42 insgesamt einstückig ausgeführt sein.

Der erste Körper 52 und der zweite Körper 54 können beispielsweise u.a. aus Kunststoff, Metall, Keramik und/oder Glas hergestellt sein, wobei die Körper 52 und 54 auch unterschiedlich aufgebaut sein können, und/oder mittels Schraub- und/oder Steck-/Rast-/Schnappverbindungen miteinander verbunden sein.

Bei der Variante von Fig. 6 sind die Fluidführungen 40 gleich dimensioniert, und haben insbesondere die gleiche Länge und im Wesentlichen überall den gleichen Querschnitt. Um dies in Verbindung damit zu erreichen, dass die Verteilungseinrichtungs-Auslässe 23 in einer Reihe angeordnet sind, wird wie folgt erreicht. Die Fluidführungen 40 verlaufen beginnend bei den Auslassöffnungen 38 der Verteilungskammer 36 zunächst im Wesentlichen geradlinig, beispielsweise in Form von Bohrungen. Diese Bereiche der Fluidführungen 40 enden bzw. öffnen sich bei den in Fig. 6 (b) mit 56a, 56b, 56c und 56d gekennzeichneten Stellen und gehen dort in Ausnehmungen 58a, 58b, 58c und 58d im ersten Körper 52 über.

Die Ausnehmungen 58a, 58b, 58c und 58c sind, wie in Fig. 6 (a) veranschaulicht, unteren Seite des ersten Körpers 52 ausgeformt. Die Ausnehmungen 58a, 58b, 58c und 58d sind so ausgeformt, dass, wenn der erste Körper 52 und der zweite Körper 54 miteinander verbunden sind, mit im Folgenden beschriebenen im zweiten Körper ausgeformten Ausnehmungen 60a, 60b, 60c und 60d jeweils einen Teil der entsprechenden Fluidführung 40 bilden.

Die Ausnehmungen 60a, 60b, 60c und 60d sind, wie in Fig. 6 (b) veranschaulicht, oberen Seite des zweiten Körpers 54 ausgeformt. Die Ausnehmungen 60a, 60b, 60c und 60d enden in Strömungsrichtung betrachtet bei Durchgängen 62a, 62b, 62c und 62d, die durch den zweiten Körper 54 zu den Fluidauslasseinrichtungs-Einlässen 30, von aus Fluid zu den Verteilungseinrichtungs-Auslässen 32 gelangen kann.

Durch die bogenartigen Verläufe der durch die Ausnehmungen 58a, 60a, 85b/60b, 58c/60c und 58d/60d gebildeten Bereiche der Fluidführungen 40 wird erreicht, dass einerseits die Fluidführungen 40 im Wesentlichen gleich dimensioniert sind, die gleiche Länge und im Wesentlichen überall den gleichen Querschnitt haben, und andererseits die Verteilungseinrichtungs-Auslässe 32 in einer Reihe angeordnet sind. Letzteres in Verbindung mit einer korrespondierenden linearen Anordnung der Fluidauslasseinrichtungen 16 und deren ersten Fluidauslasseinrichtungs-Auslässen 18 können ebenfalls linear in einer Reihe angeordnete Probenbehälter versorgt werden.

Fig. 7 veranschaulicht eine Ausführungsform einer Probenexpositionsanordnung mit einer Verteilereinrichtung 2, bei der überschüssiges Fluid rückgeführt und wiederverwendet werden kann. Von einer Fluidquelle 66 bereitgestelltes Fluid wird über eine weiter unten detaillierter beschriebene Fluidzufuhr-Selektionseinrichtung 68 einer Verteilungseinrichtung 12 zugeführt, von dieser verteilt Fluidauslasseinrichtungen 16 zugeführt. Von den Fluidauslasseinrichtungen 16 wird Fluid zur Probenexposition benötigtes Fluid Probenbehältern 6 zugeführt. Zur Probenexposition nicht benötigtes Fluid wird als überschüssiges Fluid direkt oder indirekt zur der Fluidzufuhr-Selektionseinrichtung 68 rückgeführt.

Mittels der Fluidzufuhr-Selektionseinrichtung 68 ist es selektiv möglich, Fluid der Fluidquelle 66 oder rückgeführtes Fluid der Verteilungseinrichtung 12 aber auch Mischungen davon zuzuführen, von aus der oben beschriebene Ablauf (mehrfach) wiederholt werden kann. Die Fluidzufuhr-Selektionseinrichtung 68 kann beispielsweise ein Zwei- oder Dreiwegeventil umfassen.

Zwischen den zweiten Fluidauslasseinrichtungs-Auslässen 24 und der Wahleinrichtung 68 können, jeweils optional, ein Überschussfluidbehälter 26, eine Volumenausgleichseinrichtung 70 und/oder eine Fördereinrichtung 72 angeordnet sein,
Die obigen Ausführungen bezüglich des Überschussfluidbehälters 26 gelten hier entsprechend. Die Volumenausgleichseinrichtung 70 dient dazu, die Menge an Fluid, die zur Probenexposition benötigt den Fluid Probenbehältern 6 zugeführt wird, auszugleichen. Als Volumenausgleichseinrichtung 70 kann z.B. ein Behälter mit variablem Volumen (vergleichbar mit einem Gassack oder einem Behälter mit variablem Aufnahmevolumen) verwendet werden.

Die Fördereinrichtung 72 dient dazu, einen Fluidstrom zu erzeugen, aufrechtzuhalten oder auch nur zu unterstützen, um der Verteilervorrichtung 2 Eingangsseitig einen ausreichenden Fluidstrom zuzuführen.

## Patentansprüche

1. Verteilervorrichtung für eine Probenexpositionsanordnung, die wenigstens zwei Probenbehälter (6) und eine Fluidquelle (66) für den Probenbehältern (6) zuzuführendes Fluid aufweist, umfassend:
- einer Verteilungseinrichtung (12) mit
-- einem Verteilungseinrichtungs-Einlass (34), um Fluid der Verteilervorrichtung zuzuführen,
-- jeweils einen Verteilungseinrichtungs-Auslass (32) pro Probenbehälter (6) und
-- einer Verteilungskammer (38) zwischen dem Verteilungseinrichtungs-Einlass (34) und den Verteilungseinrichtungs-Auslässen (32); und
- wenigstens eine Fluidauslasseinrichtung (16) pro Verteilungseinrichtungs-Auslass (32), wobei die wenigstens eine Fluidauslasseinrichtung (16) jeweils
-- einen Fluidauslasseinrichtungs-Einlass (30), der mit dem entsprechenden Verteilungseinrichtungs-Auslass (32) in Fluidverbindung steht,
-- einen ersten Fluidauslasseinrichtungs-Auslass (18) zur Abgabe von Fluid in den entsprechenden Probenbehälter (6), und
-- einen zweiten Fluidauslasseinrichtungs-Auslass (24) zur Abgabe von überschüssigem Fluid aufweist,
**dadurch gekennzeichnet, dass** der zweite Fluidauslasseinrichtungs-Auslass (24) dazu ausgebildet ist, um über den Fluidauslasseinrichtungs-Einlass (30) der Fluidauslasseinrichtung (16) zugeführtes Fluid, welches dem entsprechenden Probenbehälter (6) nicht zugeführt werden soll und/oder kann, abzuführen und nicht dem Probenbehälter zuzuführen.

2. Verteilervorrichtung nach Patentanspruch 1, wobei die Verteilungseinrichtung (12) zwischen der Verteilungskammer (38) und den Verteilungseinrichtungs-Auslässen (32) jeweils wenigstens eine Fluidführung (40) aufweist.

3. Verteilervorrichtung nach einem der vorherigen Patentansprüche, wobei die Verteilungskammer (38) im Wesentlichen kugelförmig oder halbkugelförmig ist.

4. Verteilervorrichtung nach einem der vorherigen Patentansprüche, wobei in der Verteilungskammer (38) eine strömungsbeeinflussende Einrichtung (42) angeordnet ist.

5. Verteilervorrichtung nach einem der Patentansprüche 2 bis 4, wobei an einer Innenseite der Verteilungskammer (38) für jede Fluidführung eine Fluidleitausnehmung (64) ausgebildet ist.

6. Verteilervorrichtung nach einem der vorherigen Patentansprüche, wobei die Verteilungseinrichtung (12) eine abnehmbare Abdeckung (48) umfasst, die wenigstens einen Teil (44) der Verteilungskammer (38) definiert.

7. Verteilervorrichtung nach einem der Patentansprüche 2 bis 6, wobei die Verteilungskammer (38) wenigstens teilweise und/oder die Fluidführungen (40) in einem ersten Körper (52) ausgebildet ist/sind.

8. Verteilervorrichtung nach einem der Patentansprüche 2 bis 7, wobei die Fluidführungen (40) wenigstens teilweise in einem zweiten Körper (54) ausgebildet sind.

9. Verteilervorrichtung nach den Patentansprüchen 7 und 8, wobei der erste Körper (52) und der zweite Körper (54) jeweils komplementäre die Fluidführungen bildende Ausnehmung (58a, 58b, 58c, 58d; 60a, 60b, 60c, 60d) aufweisen.

10. Verteilervorrichtung nach einem der Patentansprüche 2 bis 9, wobei wenigstens eine der Fluidführungen (40) gekrümmt verläuft.

11. Verteilervorrichtung nach einem der vorherigen Patentansprüche, wobei die Fluidauslasseinrichtungs-Auslässe (24) zur Abgabe von überschüssigem Fluid mit einem Überschussfluidbehälter (26) in Fluidverbindung stehen.

12. Verteilervorrichtung nach Patentanspruch 11, wobei der Überschussfluidbehälter (26) einen Auslass (28) zur Abgabe von darin vorhandenem Fluid hat.

13. Verteilervorrichtung nach einem der vorherigen Patentansprüche, ferner mit einer Fluidzufuhr-Selektionseinrichtung (68), die mit den zweiten Fluidauslasseinrichtungs-Auslässen (24) und mit dem Verteilungseinrichtungs-Einlass (34) in Fluidverbindung steht und ausgelegt ist, entweder Fluid der Fluidquelle (66) oder Fluid von den zweiten Fluidauslasseinrichtungs-Auslässen (24) oder eine Mischung von Fluid der Fluidquelle und Fluid von zweiten Fluidauslasseinrichtungs-Auslässen (24) dem Verteilungseinrichtungs-Einlass (34) zuzuführen.

14. Verteilervorrichtung nach einem der vorherigen Patentansprüche, ferner mit einer Fluidvolumenausgleichseinrichtung (74), die mit den zweiten Fluidauslasseinrichtungs-Auslässen (24) in Fluidverbindung steht.

15. Probenexpositionsanordnung mit einer Verteilervorrichtung nach einem der vorherigen Patentansprüche.

16. Probenexpositionsanordnung nach Patentanspruch 15, die wenigstens zwei Probenbehältern (6) und/oder eine Fluidquelle (66) für den Probenbehältern (6) zuzuführendes Fluid aufweist.

## Claims

1. Distribution device for a specimen exposition arrangement that comprises at least two specimen receptacles (6) and a fluid supply (66) for fluid to be supplied to the specimen receptacles (6), the distribution device comprising:
- a distribution means (12) comprising
-- a distribution means inlet (34) for supplying fluid to the distribution device,
-- for each specimen receptacle (6) a respective distribution means outlet (32), and
-- a distribution chamber (38) between the distribution means inlet (34) and the distribution means outlets (32); and
- at least one fluid outlet means (16) for each distribution means outlet (32), wherein the at least one fluid outlet means (16) respectively comprises
-- a fluid outlet means inlet (30) being in fluid connection with the respective distribution means outlet (32),
-- a first fluid outlet means outlet (18) for outputting fluid in the respective specimen receptacle (6), and
-- a second fluid outlet means outlet (24) for outputting excess fluid;
**characterized in that** the second fluid outlet means outlet (24) is adapted to conduct away fluid supplied to fluid inlet means (16) via the fluid outlet means inlet (30), which fluid shall not or cannot supplied to the respective specimen receptacle (6), and not the specimen receptacle (6).

2. Distribution device according to claim 1, wherein the distribution means (12) comprises between the distribution chamber (38) and the distribution mean outlets (32) at least one respective fluid guide (40).

3. Distribution device according to one of the preceding claims, wherein the distribution chamber (38) is essentially spherical or hemispherical.

4. Distribution device according to one of the preceding claims, wherein a flow affecting means (42) is arranged in the distribution chamber (38).

5. Distribution device according to one of the claims 2 to 4, wherein a fluid guide recess (64) is formed at an inner surface of the distribution chamber (38) for each fluid guide.

6. Distribution device according to one of the preceding claims, wherein the distribution means (12) comprises a removable cover (48) that defines at least a part (44) of the distribution chamber (38).

7. Distribution device according to one of the claims 2 to 6, wherein at least a part of the distribution chamber (38) and/or the fluid guides (40) are formed in a first body (52).

8. Distribution device according to one of the claims 2 to 7, wherein at least a part of the fluid guides (40) is formed in a second body (54).

9. Distribution device according to claims 7 and 8, wherein the first body (52) and the second body (54) comprise complementary recesses (58a, 58b, 58c, 58d; 60a, 60b, 60c, 60d) forming the fluid guides.

10. Distribution device according to one of the claims 2 to 9, wherein at least one fluid guide (40) extends in curved manner.

11. Distribution device according to one of the preceding claims, wherein the fluid outlet means outlets (24) are, for outputting excess fluid, in fluid connection with an excess fluid receptacle (26).

12. Distribution device according to claim 11, wherein the excess fluid receptacle (26) comprises an outlet (28) for outputting of fluid contained therein.

13. Distribution device according to one of the preceding claims, further comprising a fluid supply selection means (68), the fluid supply selection means (68) being in fluid connection with the second fluid outlet means outlets (24) and the distribution means inlet (34) and being adapted to supply fluid of the fluid source (66) or fluid from the second fluid outlet means outlets (24) or a mixture of fluid of the fluid source (66) and fluid from the second fluid outlet means outlets (24) to the distribution means inlet (34).

14. Distribution device according to one of the preceding claims, further comprising fluid volume balancing means (74) being in fluid connection with the second fluid outlet means outlets (24).

15. Specimen exposition arrangement comprising a distribution device according to one of the preceding claims.

16. Specimen exposition arrangement according to claim 15, comprising at least two specimen receptacles (6) and/or a fluid source (66) for fluid to be supplied to the specimen receptacles (6).

## Revendications

1. Dispositif distributeur pour un agencement d'exposition d'échantillons comprenant au moins deux récipients d'échantillon (6) et une source de fluide (66) pour un fluide à amener aux récipients d'échantillon (6), comportant
- un moyen de distribution (12) avec
-- une entrée de moyen de distribution (34) pour amener du fluide au dispositif distributeur,
-- une sortie de moyen de distribution (32) par récipient d'échantillon (6), et
-- une chambre de distribution (38) entre l'entrée de moyen de distribution (34) et les sorties de moyen de distribution (32) ; et
- au moins un moyen de sortie de fluide (16) par sortie de moyen de distribution (32), chacun desdits au moins un moyen de sortie de fluide (16) comprenant
-- une entrée de moyen de sortie de fluide (30) en communication fluidique avec la sortie de moyen de distribution (32) correspondante,
-- une première sortie de moyen de sortie de fluide (18) pour délivrer du fluide dans le récipient d'échantillon (6) correspondant et
-- une deuxième sortie de moyen de sortie de fluide (24) pour délivrer du fluide excédentaire,
**caractérisé en ce que** la deuxième sortie de moyen de sortie de fluide est conçue pour évacuer le fluide amené au moyen de sortie de fluide (16) par l'entrée de moyen de sortie de fluide (30), lequel fluide ne doit pas et/ou ne peut pas être amené au récipient d'échantillon (6) correspondant, et ne pas l'amener au récipient d'échantillon.

2. Dispositif distributeur selon la revendication 1, dans lequel le moyen de distribution (12) comprend au moins un guide de fluide (40) entre la chambre de distribution (38) et chacune des sorties de moyen de distribution (32).

3. Dispositif distributeur selon l'une des revendications précédentes, dans lequel la chambre de distribution (38) est sensiblement sphérique ou hémisphérique.

4. Dispositif distributeur selon l'une des revendications précédentes, dans lequel un moyen influençant l'écoulement (42) est disposé dans la chambre de distribution (38).

5. Dispositif distributeur selon l'une des revendications 2 à 4, dans lequel un évidement directeur de fluide (64) est formé sur un côté intérieur de la chambre de distribution (38) pour chaque guide de fluide.

6. Dispositif distributeur selon l'une des revendications précédentes, dans lequel le moyen de distribution (12) comprend un couvercle amovible (48) qui définit au moins une partie (44) de la chambre de distribution (38).

7. Dispositif distributeur selon l'une des revendications 2 à 6, dans lequel la chambre de distribution (38) est au moins partiellement formée et/ou les guides de fluide (40) sont formés dans un premier corps (52).

8. Dispositif distributeur selon l'une des revendications 2 à 7, dans lequel les guides de fluide (40) sont au moins partiellement formés dans un deuxième corps (54).

9. Dispositif distributeur selon les revendications 7 et 8, dans lequel le premier corps (52) et le deuxième corps (54) comprennent respectivement des évidements complémentaires (58a, 58b, 58c, 58d ; 60a, 60b, 60c, 60d) formant les guides de fluide.

10. Dispositif distributeur selon l'une des revendications 2 à 9, dans lequel au moins l'un des guides de fluide (40) est incurvé.

11. Dispositif distributeur selon l'une des revendications précédentes, dans lequel les sorties de moyen de sortie de fluide (24) sont en communication fluidique avec un récipient de fluide excédentaire (26) pour délivrer du fluide excédentaire.

12. Dispositif distributeur selon la revendication 11, dans lequel le récipient de fluide excédentaire (26) comprend une sortie (28) pour délivrer le fluide qui y est présent.

13. Dispositif distributeur selon l'une des revendications précédentes, comprenant en outre un moyen de sélection d'amenée de fluide (68) en communication fluidique avec les deuxièmes sorties de moyen de sortie de fluide (24) et avec l'entrée de moyen de distribution (34) et conçu pour amener soit du fluide provenant de la source de fluide (66), soit du fluide provenant des deuxièmes sorties de moyen de sortie de fluide (24), soit un mélange de fluide provenant de la source de fluide et de fluide provenant des deuxièmes sorties de moyen de sortie de fluide (24) à l'entrée de moyen de distribution (34).

14. Dispositif distributeur selon l'une des revendications précédentes, comprenant en outre un moyen de compensation de volume de fluide (74) en communication fluidique avec les deuxièmes sorties de moyen de sortie de fluide (24).

15. Agencement d'exposition d'échantillons comprenant un dispositif distributeur selon l'une des revendications précédentes.

16. Agencement d'exposition d'échantillons selon la revendication 15, qui comprend au moins deux récipients d'échantillon (6) et/ou une source de fluide (66) pour un fluide à amener aux récipients d'échantillon (6).
